# EUROPÄISCHE PATENTSCHRIFT

(11) **EP 1 391 208 B1**
(45) Veröffentlichungstag und Bekanntmachung des Hinweises auf die Patenterteilung: **26.11.2008**
(21) Anmeldenummer: 02018683.9
(22) Anmeldetag: 21.08.2002
(51) Int. Cl.: A61K 36/48, A23K 1/00

(54) **Verfahren zur Herstellung von Pflanzenextrakten mit hohem Gehalt an Isoflavonen**
Process of preparation of plant extracts with high isoflavone content
Procédé de préparation d'extraits de plantes à haute teneur en isoflavones

(43) Veröffentlichungstag der Anmeldung: 25.02.2004
(73) Patentinhaber: Cognis IP Management GmbH, 40589 Düsseldorf (DE)
(72) Erfinder: Rull Prous, Santiago, 08034 Barcelona (ES); Arias, Carmen, 08190 Sant Cugat del Vallés (ES)

(56) Entgegenhaltungen:
- LIU J ET AL: "EVALUATION OF ESTROGENIC ACTIVITY OF PLANT EXTRACTS FOR THE POTENTIAL TREATMENT OF MENOPAUSAL SYMPTOMS" JOURNAL OF AGRICULTURAL AND FOOD CHEMISTRY, AMERICAN CHEMICAL SOCIETY. WASHINGTON, US, Bd. 49, Nr. 5, 2001, Seiten 2472-2479, XP001068359 ISSN: 0021-8561
- DATABASE BIOSIS [Online] BIOSCIENCES INFORMATION SERVICE, PHILADELPHIA, PA, US; 1983 VASIL'CHENKO E A ET AL: "ANABOLIC EFFECT OF TOTAL FLAVONOIDS FROM TRIFOLIUM-PRATENSE" Database accession no. PREV198478094272 XP002227007 & RASTITEL'NYE RESURSY, Bd. 19, Nr. 4, 1983, Seiten 538-543, ISSN: 0033-9946

## Beschreibung

### Gebiet der Erfindung

Die Erfindung befindet sich auf dem Gebiet der botanischen Extrakte und betrifft ein neues Verfahren zur Herstellung von isoflavonreichen Extrakten sowie deren Verwendung in verschiedenen Gebieten.

### Stand der Technik

*Trifolium pratense*, auch unter der Bezeichnung Rotklee bekannt, ist ein zweijähriges Kraut mit roten bis purpurfarbenen Blüten, das bis zu 80 cm hoch wird und in Europa sowie Nordamerika zu Hause ist. Es enthält als Wirkstoffe vorwiegend Isoflavone und deren Glucoside, wie z.B. Daidzein, Genestein, Formononentin und Biochanin A sowie deren Glucoside, wie z.B. Ononin oder Sissostrin :

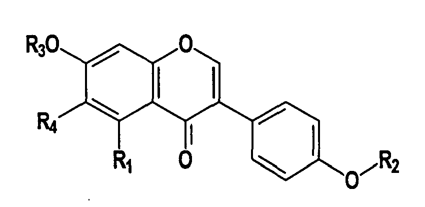

| **Isoflavonglucoside** | **R₁** | **R₂** | **R₃** | **R₄** |
|---|---|---|---|---|
| Daizidin | H | H | Glucose | H |
| Genistin | H | H | Glucose | OH |
| Ononin | H | CH₃ | Glucose | H |
| Sissostrin | H | CH₃ | Glucose | OH |

Isoflavone stellen Phytohormone mit östrogenartigen Eigenschaften dar und können daher vielfache Verwendung, beispielsweise in der Kosmetik, der Pharmazie oder aber auch als Nahrungsmittelergänzungsstoffe finden. Entsprechende isoflavonhaltige Extrakte der *Trifolium pratense* sind bereits im Handel erhältlich, da aber der Wirkstoffgehalt in der Pflanze unter 1 Gew.-% bezogen auf die Trockenmasse liegt, weisen die Konzentrate in der Regel allenfalls 20 Gew.-% Aktivsubstanz auf; für kommerzielle Anwendungen wären jedoch Konzentrationen von wenigstens 40 Gew.-% wünschenswert.

Mit der Pflanze Red Clover ist jedoch auch noch ein weiterer Nachteil verbunden. Grundsätzlich eignen sich ihre Extrakte wegen ihres Kräuteraromas als Zusatzstoff, speziell als Appetitanreger für Viehfutter. In der Praxis wird diese Idee jedoch bislang nicht realisiert, da der geringe Isoflavongehalt wegen seiner pharmakologischen Wirkung für die Tierernährung als zu hoch erachtet wird.

Somit scheitert die kommerzielle Nutzung von Extrakten der *Trifolium pratense* für Kosmetik, Pharmazie und Nahrungsmittelergänzungsstoffe an dem zu niedrigen Isoflavongehalt, während dieser andererseits zu hoch ist, um die Stoffe in der Tierernährung einzusetzen.

Die komplexe Aufgabe der vorliegenden Erfindung hat folglich darin bestanden, ein Verfahren zur Verfügung zu stellen, welches ausgehend von der Pflanze *Trifolium pratense* gleichzeitig zwei Probleme löst, nämlich zum einen ein Zwischenprodukt zur Verfügung stellt, welches praktisch frei von Isoflavonen ist und in der Tierernährung eingesetzt werden kann und zum anderen Isoflavonkonzentrate, die wegen ihrer östrogenartigen Wirkung zur Herstellung von kosmetischen oder pharmazeutischen Zubereitungen, aber auch als Nahrungsmittelergänzungsstoffe eingesetzt werden können.

### Beschreibung der Erfindung

Gegenstand der Erfindung ist ein Verfahren zur Herstellung von Extrakten des *Trifolium pratense* mit einem Gehalt von mindestens 40 Gew.-% Isoflavonen - bezogen auf den Feststoffgehalt - bei dem man
(a) die Pflanzen mit Wasser extrahiert und eine wässrige von Isoflavonen praktisch freie Phase IB und einen mit Isoflavonen angereicherten Rückstand IA gewinnt,
(b) den Rückstand IA mit Methanol extrahiert, den Extrakt vom Methanol befreit und so einen Rückstand II gewinnt, welcher mindestens 10 Gew.-% Isoflavone enthält,
(c) den Rückstand II mit Aceton und/oder aliphatischen Alkoholen mit 2 bis 5 Kohlenstoffatomen wie beispielsweise Ethanol oder Isopropylalkohol extrahiert, den Extrakt vom Lösungsmittel befreit und so einen Rückstand III gewinnt, der mindestens 30 Gew.-% Isoflavone enthält,
(d) den Rückstand III mit Ethanol extrahiert, den Extrakt vom Ethanol befreit und so einen Rückstand IV gewinnt, der mindestens 40 Gew.-% Isoflavone enthält, sowie gegebenenfalls
(e) den Rückstand IV mit Ethylacetat extrahiert, den Extrakt vom Ethylacetat befreit und so einen Rückstand V gewinnt, der mindestens 80, vorzugsweise 80 bis 100 Gew.-% Isoflavone enthält.

Überraschenderweise wurde gefunden, dass mit Hilfe einer genau abgestimmten Kaskade von Lösungsmitteln unterschiedlicher Polarität der Isoflavongehalt in den einzelnen Extrakten schrittweise gesteigert werden kann, bis schließlich Produkte in einer Reinheit von mindestens 40, vorzugsweise mindestens 60, insbesondere mindestens 70 und besonders bevorzugt mindestens 80 oder sogar praktisch 100 % Gew.-ige Produkte erhalten werden. Schon bei der ersten wässrigen Extraktion fällt ein Trockenrückstand an, der praktisch frei von Isoflavonen ist und damit wegen seines Kräuteraromas bedenkenlos als Appetitanreger in der Tierernährung eingesetzt werden kann. Die Isoflavonkonzentrate, die in unterschiedlicher Reinheit als Zwischenprodukte erhalten werden, können jedes für sich für die Herstellung von kosmetischen oder pharmazeutischen Zubereitungen oder als Nahrungsmittelergänzungsstoffe dienen.

### Extraktion

Die Herstellung der Extrakte kann in an sich bekannter Weise erfolgen, d.h. beispielsweise durch wässrigen, alkoholischen, wässrig-alkoholischen oder organischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Die vorliegenden Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte können als solche eingesetzt werden, es ist jedoch ebenfalls möglich, das Lösungsmittel durch Trocknung, insbesondere durch Sprüh- oder Gefriertrocknung vollständig zu entfernen.

### Gewerbliche Anwendbarkeit

Im ersten Schritt des Verfahrens wird eine wässrige Phase IB erhalten, in der etwa 20 Gew.-% der wasserlöslichen Stoffe, jedoch praktisch keine Isoflavone mehr enthalten sind. Ein weiterer Gegenstand der Erfindung betrifft daher die Verwendung dieser Phase bzw. des daraus durch Trocknung erhältlichen Rückstandes als Zusatzstoff, speziell als Appetitanreger für die Tierernährung. Die übrigen Isoflavonkonzentrate, die als Zwischenprodukte bzw. als Endprodukt anfallen, können im Sinne weiterer Gegenstände der Erfindung zur Herstellung von kosmetischen oder pharmazeutischen Zubereitungen sowie als Nahrungsmittelergänzungsstoffe verwendet werden.

### Beispiele

### Beispiel 1

In einem Reaktor wurden 5 kg *Triflolium pratense* vorgelegt und bei 20 °C mit Wasser gewaschen, bis etwa 20 Gew.-% der wasserlöslichen Substanzen in die wässrige Phase (IB) übergegangen waren. Diese wurde vom Rückstand IA abgetrennt und separat getrocknet und als Tiernahrungszusatzstoff verwertet. Der Rückstand IA wurde hingegen getrocknet und bei einer Temperatur von 70 °C mit 10 l 60 Gew.-%igem wässrigem Methanol extrahiert. Anschließend wurde das Lösungsmittel abgezogen, wobei ein Trockenrückstand II erhalten wurde, welcher 12 Gew.-% Isoflavone enthielt. Der Rückstand II wurde sodann bei einer Temperatur von 20 °C mit 10 l Aceton extrahiert. Anschließend wurde wieder das Lösungsmittel abgetrennt, wobei ein neuer Trockenrückstand III erhalten wurde, welcher 35 Gew.-% Isoflavone enthielt. Der Rückstand III wurde sodann bei einer Temperatur von 20 °C mit 10 l Ethanol extrahiert. Anschließend wurde das Lösungsmittel wieder abgetrennt, wobei ein Trockenrückstand IV erhalten wurde, welcher 50 Gew.-% Isoflavone aufwies.

### Beispiel 2

Der Trockenrückstand IV aus Beispiel 1 wurde bei 20 °C mit 10 l Ethylaceat extrahiert. Anschließend wurde das Lösungsmittel abgetrennt, wobei ein Trockenrückstand V erhalten wurde, welcher 85 Gew.-% Isoflavone aufwies.

### Beispiel 3

Beispiel 2 wurde wiederholt, die Extraktion des Trockenrückstands II jedoch mit einer 1:1-Mischung aus Aceton und Isopropylalkohol durchgeführt. Der so erhaltene Trockenrückstand III wies einen Isoflavongehalt von 40 Gew.-% auf. Das angereicherte Zwischenprodukt wurde wie oben beschrieben weiter verarbeitet, wobei schließlich ein Trockenrückstand V erhalten wurde, welcher einen Isoflavongehalt von 92 Gew.-% aufwies.

## Patentansprüche

1. Verfahren zur Herstellung von Extrakten des *Trifolium pratense* mit einem Gehalt von mindestens 40 Gew.-% Isoflavonen - bezogen auf den Feststoffgehalt - bei dem man
(a) die Pflanzen mit Wasser extrahiert und eine wässrige von Isoflavonen praktisch freie Phase IB und einen mit Isoflavonen angereicherten Rückstand IA gewinnt,
(b) den Rückstand IA mit Methanol extrahiert, den Extrakt vom Methanol befreit und so einen Rückstand II gewinnt, welcher mindestens 10 Gew.-% Isoflavone enthält,
(c) den Rückstand II mit Aceton und/oder aliphatischen Alkoholen mit 2 bis 5 Kohlenstoffatomen extrahiert, den Extrakt vom Lösungsmittel befreit und so einen Rückstand III gewinnt, der mindestens 30 Gew.-% Isoflavone enthält, und
(d) den Rückstand III mit Ethanol extrahiert, den Extrakt vom Ethanol befreit und so einen Rückstand IV gewinnt, der mindestens 40 Gew.-% Isoflavone enthält.

2. Verfahren nach Anspruch 1, **dadurch gekennzeichnet, dass** man den Rückstand IV mit Ethylacetat extrahiert, den Extrakt vom Ethylacetat befreit und so einen Rückstand V erhält, der 80 bis 100 Gew.-% Isoflavone enthält.

3. Verfahren nach den Ansprüchen 1 und/oder 2, **dadurch gekennzeichnet, dass** die Isoflavonkonzentrate Daidzein, Genestein, Formononentin und Biochanin A enthalten.

4. Verwendung des der wässrigen Phase IB bzw. des daraus gewonnenen Rückstandes, erhältlich nach dem Verfahrens des Anspruchs 1 für die Tierernährung.

5. Verwendung der Isoflavonkonzentrate, erhältlich nach den Verfahren der Ansprüche 1 und/oder 2 zur Herstellung von kosmetischen Zubereitungen.

6. Verwendung der Isoflavonkonzentrate, erhältlich nach den Verfahren der Ansprüche 1 und/oder 2 zur Herstellung von pharmazeutischen Zubereitungen.

7. Verwendung der Isoflavonkonzentrate, erhältlich nach den Verfahren der Ansprüche 1 und/oder 2 als Nahrungsmittelergänzungsstoffe.

## Claims

1. Process for the preparation of extracts of *Trifolium pratense* with a content of at least 40% by weight of isoflavones, based on the solids content, where
(a) the plants are extracted with water and an aqueous, virtually isoflavone-free phase IB and an isoflavone-enriched residue IA are obtained,
(b) the residue IA is extracted with methanol, and the extract is freed from the methanol, giving a residue II which contains at least 10% by weight of isoflavones,
(c) the residue II is extracted with acetone and/or aliphatic alcohols having 2 to 5 carbon atoms and the extract is freed from the solvent, giving a residue III which contains at least 30% by weight of isoflavones, and
(d) the residue III is extracted with ethanol and the extract is freed from the ethanol, giving a residue IV which contains at least 40% by weight of isoflavones.

2. Process according to Claim 1, **characterized in that** the residue IV is extracted with ethyl acetate and the extract is freed from the ethyl acetate, giving a residue V which contains 80 to 100% by weight of isoflavones.

3. Process according to Claims 1 and/or 2, **characterized in that** the isoflavone concentrates contain daidzein, genestein, formononentin and biochanin A.

4. Use of the aqueous phase IB or the residue obtained therefrom, obtainable by the process of Claim 1, for animal nutrition.

5. Use of the isoflavone concentrates obtainable by the processes of Claims 1 and/or 2 for the preparation of cosmetic products.

6. Use of the isoflavone concentrates obtainable by the processes of Claims 1 and/or 2 for the preparation of pharmaceutical products.

7. Use of the isoflavone concentrates obtainable by the processes as of Claims 1 and/or 2 as food supplements.

## Revendications

1. Procédé de préparation d'extraits de *Trifolium pratense* ayant une teneur en isoflavones d'au moins 40 % en poids par rapport à la teneur en matière solide, selon lequel :
(a) on extrait les plantes avec de l'eau et on obtient une phase aqueuse IB pratiquement exempte d'isoflavones et un résidu IA enrichi en isoflavones,
(b) on extrait le résidu IA avec du méthanol, on libère l'extrait du méthanol et on obtient ainsi un résidu II qui renferme au moins 10 % en poids d'isoflavones,
(c) on extrait le résidu II avec de l'acétone et/ou des alcools aliphatiques ayant de 2 à 5 atomes de carbone, on libère l'extrait du solvant, et on obtient ainsi un résidu III qui renferme au moins 30 % en poids d'isoflavones, et
(d) on extrait le résidu III avec de l'éthanol, on libère l'extrait de l'éthanol, et on obtient ainsi un résidu IV qui renferme au moins 40 % en poids d'isoflavones.

2. Procédé selon la revendication 1,
**caractérisé en ce qu'**
on extrait le résidu IV avec de l'acétate d'éthyle, on libère l'extrait de l'acétate d'éthyle et on obtient ainsi un résidu V qui renferme 80 % à 100 % en poids d'isoflavones.

3. Procédé selon les revendications 1 et/ou 2,
**caractérisé en ce que**
les concentrés d'isoflavones renferment de la daidezeine, de la genes-teine de la formononetine et de la biochanine A.

4. Utilisation du résidu de la phase aqueuse IB ou issu de celui-ci pouvant être obtenu par la mise en oeuvre du procédé selon la revendication 1 pour l'alimentation animale.

5. Utilisation des concentrés d'isoflavones pouvant être obtenus par la mise en oeuvre du procédé selon les revendications 1 et/ 2 pour l'obtention de préparations cosmétiques.

6. Utilisation des concentrés d'isoflavones pouvant être obtenus par la mise en oeuvre du procédé selon les revendications 1 et/ou 2 pour l'obtention de préparations pharmaceutiques.

7. Utilisation des concentrés d'isoflavones pouvant être obtenus par la mise en oeuvre du procédé selon les revendications 1 et/ou 2 en tant que compléments alimentaires.
